# NEUE EUROPÄISCHE PATENTSCHRIFT

(11) **EP 0 556 651 B2**
(45) Veröffentlichungstag und Bekanntmachung des Hinweises auf die Entscheidung über den Einspruch: **03.11.1999**
(45) Hinweis auf die Patenterteilung: 05.07.1995
(21) Anmeldenummer: 93101719.8
(22) Anmeldetag: 04.02.1993
(51) Int. Cl.: C12N 15/12, C07K 14/435

(54) **Humane neuronale Beta-Untereinheiten spannungsabhängiger Calciumkanäle und deren Verwendung**
Human neuronal beta-subunits of voltage-dependent calcium channels and use of the same
Sous-unités bêta des canaux calcium neuronaux humains, dépendant du voltage et leur utilisation

(30) Priorität: 17.02.1992 DE 4204716; 06.07.1992 DE 4222126
(43) Veröffentlichungstag der Anmeldung: 25.08.1993
(73) Patentinhaber: BAYER AG, 51368 Leverkusen (DE)
(72) Erfinder: Spreyer, Peter, Dr., W-4000 Düsseldorf 1 (DE); Unterbeck, Axel, Dr., c/o Miles Inc., West Haven (US)

(56) Entgegenhaltungen:
- SCIENCE Bd. 245, 8. September 1989, WASHINGTON Seiten 1115 - 1118 P. RUTH ETAL. 'Primary structure of beta subunit ...'
- SCIENCE Bd. 253, 27. September 1991, WASHINGTON Seiten 1553 - 1557 D. SINGER ETAL. 'The role of the subunits in the function of calcium channel'
- J. BIOLOG. CHEM. Bd. 267, Nr. 32, 15. November 1992, ROCKVILLE PIKE Seiten22967 - 22972 P. A. POWERS ET AL. 'Skeletal muscle and brain isoforms of abeta-subunit of human voltage dependent calcium channels are encoded by asingle gene'
- NATURE Bd. 328, 23. Juli 1987, LONDON Seiten 313 - 318 T. TANABE ET AL.'Primary structure of the receptor for calcium channel blckers from skeletalmuscle'

## Beschreibung

Die vorliegende Erfindung betrifft humane neuronale Beta-Untereinheiten spannungsabhängiger Calciumkanäle und deren Verwendung in Screening-Verfahren zum Auffinden von Arzneimitteln.

Calcium-Ionen haben in jedem biologischen System vielfältige Funktionen. Die zelluläre Calciumhomöostase spielt speziell für die Physiologie von Nervenzellen eine wesentliche Rolle. Die intrazelluläre Calcium-Konzentration beträgt etwa 0,1 µM gegenüber 1 mM außerhalb der Nervenzelle. Die Regulation dieses starken Konzentrationsgefälles (x 10.000) erfolgt primär durch spannungsabhängige Calcium-Kanäle (VOCC), die von bestimmten Calcium-Antagonisten blockiert werden können. Während einer cerebralen Ischämie (Hirnschlag) wird die Calciumhomöostase in Neuronen des betroffenen Infarktgebietes erheblich verändert. Die spannungsabhängigen Calcium-Kanäle werden durch anhaltende Membrandepolarisationen im geöffneten Zustand gehalten, welches einen massiven Einstrom von Calcium-Ionen zur Folge hat. Die intrazelluläre Calcium-Konzentration steigt dabei um das 1000fache an. Der hohe Überschuß an Calcium aktiviert durch die Bindung an Calmodulin verschiedene Calcium/Calmodulin anhängige zelluläre Enzymsysteme, wie Kinasen, Proteasen und Phospholipasen, die zusammen, bei anhaltender Aktivierung, zu irreversiblen Nervenzellschädigungen führen.

Ein therapeutischer Ansatz zur Neuroprotektion bei cerebraler Ischämie ist die reversible Blockierung des massiven Calcium-Einstroms in die Nervenzelle. Die spannungsabhängigen neuronalen Calcium-Kanäle sind hierbei ein geeigneter pharmakologischer Angriffspunkt. VOCCs existieren in verschiedenen Muskelzellen (Gefäß-, Herz- und Skelettmuskel), Neuronen und sekretorischen Zellen mit gewebespezifischen physiologischen Eigenschaften.

Elektrophysiologische Untersuchungen (Tsien et al., 1988, Trends in Neurol.Sci 11:431-438) deuten auf mindestens drei verschiedene Typen von VOOCs hin (L-, N- und T-Kanäle). Die 1,4-Dihydropyridine (DHPs) sind potente Blocker der L-Typ Calcium-Kanäle, die sowohl in Muskelzellen als auch in Nervenzellen zu finden sind. Der Kaninchen-Skelettmuskel Dihydropyridin Rezeptor ist biochemisch charakterisiert und kloniert worden (Tanabe et al., 1987, Nature 328: 313-318). Die Primärsequenz dieser α1 UE des VOCC konnte von den cDNA Daten abgeleitet werden und ist konsistent mit einem 212 kD Transmembranprotein mit fünf N-Glycosylierungsstellen und sieben möglichen Phosphorylierungsstellen. Das Protein enthält vier untereinander ähniche Transmembrandomänen mit jeweils sechs - vermutllch α-helikalen - membrangängigen Segmenten (S1-S6). Jeweils das vierte Transmenbransegment (S4) jeder Domäne enthält ein geordnetes Muster an positiven Ladungen (Lys. Arg), welcher den Spannungssensor des Calcium-Kanals bilden können. Die Struktur dieser klonierten α1 UE ist konsistent mit einer Ionen leitenden, spannungsgesteuerten Einheit des DHP-sensitiven Calcium-Kanals.

Neben der Alpha-1 Untereinheit, die den eigentlichen Calciumkanal bildet, sind vier weitere Proteine am Aufbau des vollständigen Kanalkomplexes beteiligt, die als Alpha-2, Beta, Gamma und Delta Untereinheiten bezeichnet werden.

Neuere Untersuchungen zeigen, daß insbesondere die Beta Untereinheit wichtige Parameter der Kanalfunktion beeinflußt: Wenn die α₁- und β-UE in vitro zusammen exprimiert werden, verändert sich der Stromfluß, die Aktivierungs- und Inaktivierungskinetik des Kanalkomplexes sowie die Bindungsaffinität für Dihydropyridine (Singer et al., 1991, Science 253: 1553; Lacerda et al., 1991, Nature 352: 527; Varadi et al., 1991, Nature 352: 159).

Die 100 93/040 803 eine - Referenz unter Art. 54(3) EPU beschreibt Kalziumkanäle, die sich von dem hier beanspruchten Kanal unterscheiden.

Für die Rekonstitution einer physiologisch relevanten Kanalstruktur durch Genexpression in Eukaryontenzellen ist daher die Verfügbarkeit vollständiger cDNA-Klone der Beta Untereinheit von großer Bedeutung.

Mit Hilfe eines Oligonukleotids, das von der DNA-Sequenz der Beta Untereinheit aus dem Skelettmuskel des Kaninchens (Ruth et al., 1989, Science 245: 1115) abgeleitet wurde, konnten 3 verschiedene cDNA-Typen isoliert werden, welche humane, neuronale Beta Untereinheiten kodieren. Die klonierten Beta Untereinheiten sollen zusammen mit bereits vorhandenen Subtypen neuronaler Alpha-1 Klone in transformierten Tierzellen (z.B. cos Zellen, Maus L Zellen, CHO Zellen etc.) exprimiert werden (Gluzman, 1981, Cell 23: 175 und Chen et al., 1987, Mol. Cell.Biol.7: 2745-2752). Diese Konstrukte werden in Bindungstests und/oder funktionellen Testsytemen eingesetzt, die zur Auffindung neuer, Subtypspezifischer Liganden neuronaler Calciumkanäle dienen.

Weiterhin sollen mit diesen rekombinanten Zellsystemen funktionale Calcium-Flux Assays entwickelt werden, mit deren Hilfe spezifische Liganden auf ihre agonistische bzw. antagonistische Wirkung überprüft werden können. Der Unterschied und Hauptvorteil dieser rekombinanten Assays, im Vergleich zu herkömmlichen Assays (Himmembranpräparationen, Zellinien), liegt in der Reinheit des Rezeptors/Kanal Präparates, da ausschließlich der rekombinant exprimierte neuronale Calcium-Kanal Subtyp in beliebiger Anzahl auf einer Tierzell-Oberfläche präsent ist. Dies ist eine essentielle Voraussetzung für die Selektion spezifischer neuronaler Liganden, die möglichst keine Wirkung auf Calcium-Kanäle nicht neuronaler Gewebetypen zeigen sollen.

Nachfolgend sind einige Beispiele für die Anwendung der oben beschriebenen rekombinanten Screeningassays aufgeführt.

### 1. Rezeptor-Bindungs-Test

Die mit humanen Calcium-Kanal Subtypen transformierten Tierzellen (Beispiel: siehe oben) können kultiviert und zur Präparation von Membranen eingesetzt werden. Diese Membranpräparatioren können für Bindungsstudien mit verschiedenen radioaktiv markierten Substanzklassen (Beisp. 1-5) mm Screening neuer Liganden (Kompetitionstest) eingesetzt werden. Beispiele für bekannte Calcium-Kanal bindende Substanzen sind:
1. Phenylalkylamine,
2. Benzothiazepine,
3. Dihydropyridine,
4. Bisphenylbutylpiperidine,
5. Omega Conotoxine.

### 2. Calcium-45-Flux-Test

Die Zellmembranen von Kulturzellen, die mit humanen Calcium-Kanal Subtypen transformiert wurden, können mit Kalium-Ionen oder mit Alkaloiden wie z.3. Veratridin depolarisiert werden. Membrandepolarisation führt zur Öffnung von Calcium-Kanälen, was einen Einstrom (Flux) vcn Calcium-Ionen in die Zellen zur Folge hat. Dieser spannungsabhängige Calcium-Eirstrom kann mit radioaktiv markiertem Calcium (⁴⁵Ca) gemessen werden (Beisp.: Messing et al., 1985, J. Pharmacology and Exp. Therapeutics 235: 407-411) und zum funktionellen Testen/Screening von Calcium-Kanal Antagonisten oder Agonisten eingesetzt werden.

### 3. Fura-2 Test

Humane Calcium-Kanal exprimierende Tierzellen (s.o.) können in Gegenwart von Calcium sensitiven, fluoreszierenden Farbstoffen (z.B. Fura-2 oder Fluoro-3) für Messungen der intrazellulären Calcium-Konzentration nach Öffnung und Blockierung der Calcium-Kanäle eingesetzt werden (Beisp.: Rosario et al. 1989, Neurosci. 29, 735-747). Die Änderung der intrazellulären Calcium-Konzentration kann dabei fluorimetrisch (spektrophotometrisch) gemessen werden. Dieses rekombinante Zellsystem kann als funktioneller Test für das Auffinden Subtyp-spezifischer Calcium-Kanal Liganden (Agonisten und Antagonisten eingesetzt werden.

### 4. Elektrophysiologie

Die durch Membrandepolarisation erzeugten Calcium-Ströme können elektrophysiologisch gemessen werden (Beisp.: Carbone et al. 1990, Pflügers Arch., 416: 170-179). Die Wirkung von potentiellen Calcium-Kanal Antagonisten oder Agonisten kann direkt an humanen Calcium-Kanälen mit Hilfe der rekombinanten Tierzelllinien (s.o.) physikalisch gemessen und pharmakologisch charakterisiert werden.

### 5. Indirekte Meßmethoden

Viele zelluläre Prozesse werden von der intrazellulären Calcium-Ionen Konzentration reguliert (z.B. Rezeptor mediierte Signalübertragung, verschiedene Enzymreaktioren, wie z.B. Phosphorylierung, Dephosprorylierungen, Neurotransmitter-Freisetzung, Ca-abhängige Genregulation, etc.). Einige dieser biochemischen Reaktionen sind mit einem spezifischen Assay meßbar. In einem rekombinante Calcium-Kanäle exprimierenden Zellsystem kann somit indirekt (physiologisch) die Wirkung von Calcium-Kanal Modulatoren auf Calcium abhängige Zellvorgänge erfaßt werden (Beisp.: Zernig et al., 1986, Eur. J. Pharmacol. 128, 221-229).

Zusätzlich können durch gezielte Mutagenesen eingeführte Veränderungen, wie z.B. Punkimutationen, Insertionen, Deletionen, Austausch von DNA-Segmenten verschiedener Calciumkanal-Subtypen, direkte Auswirkungen auf physiologische Vorgänge erfaßt werden (Beisp.: Yool and Schwarz, 1991, Nature 349: 700-704).

### Klonierungsstrategie

### 1. Screenen der cDNA Bank

### 1.1. Ausplattieren der cDNA Bank und Prozessieren der Nitrozellulosefilter

Das Plattieren der cDNA Bank (humaner Hippocampus in Lambda ZAPII, Fa. Stratagene Inc., La Jolla, CA, USA; Kat.-Nr. 936205) und der Nitrozellulosefilter erfolgte gemäß den Angaben der Hersteller oder nach Sambrook et al., 1989, Molecular Cloning, A laboratory manual, Cold Spring Harbor Laboratory Press, New York, N.Y., USA.

### 1.2 Hybridisierungsproben

Als primäre Hybridisierungsprobe wurde ein synthetisches Antisense Oligonukleotid verwendet, das zu einem 40 Basen langen Fragment (Pos. 361-400) der DNA Sequenz der Beta Untereinheit aus dem Skelettmuskel des Kaninchens komplementär ist: 5'-CTTAAGGCTTCCCGGTCCTCCTCCAGGGAGACATCAGAGT-3' Die genannte DNA Sequenz ist über die EMBL Datenbank unter der Access-Nr. M25817 zugänglich.

Mit Hilfe dieses Oligonukleotids wurde das 1,9 kB lange cDNA Fragment HB 26 aus der o.g. cDNA Bank isoliert. Dieses wurde in allen weiteren Screening Experimenten als Hybridisierungsprobe eingesetzt.

### 1.3 Markieren der Hybridisierungsproben mit radioaktiven DNA Vorläufern

Oligonukleotide wurden mit ³²P-dCTP enzymatisch markiert ("DNA Tailing Kit", Boehringer Mannheim GmbH, Postfach 310120, D-6800 Mannheim: Kat.-Nr. 1028707).

Die Markierung von cDNA Fragmenten erfolgt mit ³²P-dCTP unter Verwendung des "Random Primed Labeling Kits" (Boehringer Mannheim GmbH. Kat.-Nr. 10004760).

### 1.4 Hybridisierungs- und Waschbedingungen

### 1.4.1 Oligonukleotide

Die Nitrozellulosefilter wurden mit der radioaktiv markierten Hybridsierungsprobe über Nacht bei 42°C in folgender Lösung hybridisiert:
5 x Denhardt's Lösung
5 x SSC
50 µg/ml Heringsperma DNA
50 mmol/l Na-Phosphat
1 mmol/l Na-pyrophosphat
60 µg ml ATP

Das Waschen der Filter erfolgte in 2xSSC, 0,1 % SDS bei 55 °C.

### 1.4.2. cDNA-Fragmente

Die Hybridisierung der Nitrozellulosefilter mit der radioaktiv markierten Probe erfolgt über Nacht bei 42°C in der unter 1.4.1. genannten Lösung, die jedoch mit 50 % Formamid angesetzt wurde.

Das Waschen der Filter erfolgte in 0.2xSS, 0,1 % SDS bei 55°C.

Danach wurde Kodak X-Omat AR Röntgenfilm für unterschiedliche Zeiten bei -80°C mit Verstärkerfotien durch die Fiiter belichtet.

### 2. Isolieren der Lambda Phagen, Subklonieren und Sequenzieren der cDNA Inserts

### 2.1. Überführen der cDNA Inserts in Plasmide

Die cDNA Inserts aus positiven Lambda-ZAPII Phagen wurden nach einem Protokoll des Herstellers (Stratagene) mittels eines fl-abgeleiteten Heiferphagens ausgenommen und in die Plasmidform überführt.

### 2.2. Größenbestimmung und Sequenzanalyse der cDNA Inserts

Aus XL1-Blue Zellen, die ein rekombinantes pBluescript Plasmid trugen, wurde Plasmid DNA präpariert (Sambrook, J., et al., (1989) in: Molecular cloning, A laboratory mancal, Cold Spring Harbor Laboratory Press, New York, N.Y.) und jeweils 0,5 µg dieser DNA mit dem Restriktionsenzym EcoRI behandelt. Aus Anzahl und Größe der entstandenen DNA Fragmente konnte die Gesamtlänge der insertierten cDNA abgeleitet werden. Die Nukleotidsequenz der vorhandenen cDNA wurde mit SEQUENASE (USB, Cleveland, Ohio, USA) nach der Methode von Sanger an doppelsträngiger DNA ermittelt.

### 3. Beschreibung der bisher isolierten cDNA Klone für humane neuronale Beta-Untereinheiten

### 3.1. HBB2: Länge 1,8 kB

Dieser Klon repräsentiert einen weiteren Subtyp humaner neuronaler Beta Untereinheiten mit 74 % Homologie zur Beta Untereinheit aus Kaninchenmuskel. HB28t7 ist eine Teilsequenz des gesamten Klons HBB2.

Das Sequenzprotokoll HBB2 umfaßt 1830 bp und enthält die gesamte kodierende Region.

Sequenzprotokoll - Sequenz-Nr. 29178-2 - Int. Code: HBB2 -
Länge: 1830 bp - Typ: DNA + Aminosäure

10 30 50
CCCAAGCTCGAAATTAACCCTCACTAAAGGGAACAAAAGCTGGAGCTCCACCGCGGTGGC

70 90 110
GGCCGCTCTAGAACTAGTGGATCCCCCGGGCTGCAGGAATTCCCGGACTCCCCCATGTAT
MetTyr

130 150 170
GACGACTCCTACGTGCCCGGGTTTGAGGACTCGGAGGCGGGTTCAGCCGACTCCTACACC
AspAspSerTyrValProGlyPheGluAspSerGluAlaGlySerAlaAspSerTyrThr

190 210 230
AGCCGCCCATCTCTGGACTCAGACGTCTCCCTGGAGGAGGACCGGGAGAGTGCCCGGCGT
SerArgProSerLeuAspSerAspValSerLeuGluGluAspArgGluSerAlaArgArg

250 270 290
GAAGTAGAGAGCCAGGCTCAGCAGCAGCTCGAAAGGGCCAAGCACAAACCTGTGGCATTT
GluValGluSerGlnAlaGlnGlnGlnLeuGluArgAlaLysHisLysProValAlaPhe

310 330 350
GCGGTGAGGACCAATGTCAGCTACTGTGGCGTACTGGATGAGGAGTGCCCAGTCCAGGGC
AlaValArgThrAsnValSerTyrCysGlyValLeuAspGluGluCysProValGlnGly

370 390 410
TCTGGAGTCAACTTTGAGGCCAAAGATTTTCTGCACATTAAAGAGAAGTACAGCAATGAC
SerGlyValAsnPheGluAlaLysAspPheLeuHisIleLysGluLysTyrSerAsnAsp

430 450 470
TGGTGGATCGGGCGGCTAGTGAAAGAGGGCGGGGACATCGCCTTCATCCCCAGCCCCCAG
TrpTrpIleGlyArgLeuValLysGluGlyGlyAspIleAlaPheIleProSerProGln

490 510 530
CGCCTGGAGAGCATCCGGCTCAAACAGGAGCAGAAGGCCAGGAGATCTGGGAACCCTTCC
ArgLeuGluSerIleArgLeuLysGlnGluGlnLysAlaArgArgSerGlyAsnProSer

550 570 590
AGCCTGAGTGACATTGGCAACCGACGCTCCCCTCCGCCATCTCTAGCCAAGCAGAAGCAA
SerLeuSerAspIleGlyAsnArgArgSerProProProSerLeuAlaLysGlnLysGln

610 630 650
AAGCAGGCGGAACATGTTCCCCCGTATGACGTGGTGCCCTCCATGCGGCCTGTGGTGCTG
LysGlnAlaGluHisValProProTyrAspValValProSerMetArgProValValLeu

670 690 710

Sequenzprotokoll - Sequenz-Nr. 29178-2 - Int. Code: HBB2 -
Länge: 1830 bp - Typ: DNA + Aminosäure

GTGGGACCCTCTCTGAAAGGTTATGAGGTCACAGACATGATGCAGAAGGCTCTCTTCGAC
ValGlyProSerLeuLysGlyTyrGluValThrAspMetMetGlnLysAlaLeuPheAsp
730 750 770
TTCCTCAAACACAGATTTGATGGCAGGATCTCCATCACCCGAGTCACAGCCGACCTCTCC
PheLeuLysHisArgPheAspGlyArgIleSerIleThrArgValThrAlaAspLeuSer
790 810 830
CTGGCAAAGCGATCTGTGCTCAACAATCCGGGCAAGAGGACCATCATTGAGCGCTCCTCT
LeuAlaLysArgSerValLeuAsnAsnProGlyLysArgThrIleIleGluArgSerSer
850 870 890
GCCCGCTCCAGCATTGCGGAAGTGCAGAGTGAGATCGAGCGCATATTTGAGCTGGCCAAA
AlaArgSerSerIleAlaGluValGlnSerGluIleGluArgIlePheGluLeuAlaLys
910 930 950
TCCCTGCAGCTAGTAGTGTTGGACGCTGACACCATCAACCACCCAGCACAGCTGGCCAAG
SerLeuGlnLeuValValLeuAspAlaAspThrIleAsnHisProAlaGlnLeuAlaLys
970 990 1010
ACCTCGCTGGCCCCCATCATCGTCTTTGTCAAAGTGTCCTCACCAAAGGTACTCCAGCGT
ThrSerLeuAlaProIleIleValPheValLysValSerSerProLysValLeuGlnArg
1030 1050 1070
CTCATTCGCTCCCGGGGGAAGTCACAGATGAAGCACCTGACCGTACAGATGATGGCATAT
LeuIleArgSerArgGlyLysSerGlnMetLysHisLeuThrValGlnMetMetAlaTyr
1090 1110 1130
GATAAGCTGGTTCAGTGCCCACCGGAGTCATTTGATGTGATTCTGGATGAGAACCAGCTG
AspLysLeuValGlnCysProProGluSerPheAspValIleLeuAspGluAsnGlnLeu
1150 1170 1190
GAGGATGCCTGTGAGCACCTGGCTGAGTACCTGGAGGTTTACTGGCGGGCCACGCACCAC
GluAspAlaCysGluHisLeuAlaGluTyrLeuGluValTyrTrpArgAlaThrHisHis
1210 1230 1250
CCAGCCCCTGGCCCCGGACTTCTGGGTCCTCCCAGTGCCATCCCCGGACTTCAGAACCAG
ProAlaProGlyProGlyLeuLeuGlyProProSerAlaIleProGlyLeuGlnAsnGln
1270 1290 1310
CAGCTGCTGGGGGAGCGTGGCGAGGAGCACTCCCCCCTTGAGCGGGACAGCTTGATGCCC
GlnLeuLeuGlyGluArgGlyGluGluHisSerProLeuGluArgAspSerLeuMetPro
1330 1350 1370
TCTGATGAGGCCAGCGAGACGTCCCGCCAAGCCTGGACAGGATCTTCACAGCGTACGTCC

Sequenzprotokoll - Sequenz-Nr. 29178-2 - Int. Code: HBB2 -
Länge: 1830 bp - Typ: DNA + Aminosäure

SerAspGluAlaSerGluThrSerArgGlnAlaTrpThrGlySerSerGlnArgThrSer

1390 1410 1430

CGCCACCTGGAGGAGGACTATGCAGATGCCTACCAGGACCTGTACCAGCCTCACCGCCAA

ArgHisLeuGluGluAspTyrAlaAspAlaTyrGlnAspLeuTyrGlnProHisArgGln

1450 1470 1490

CACACCTCGGGGCTGCCTAGTGCTAACGGGCATGACCCCCAAGACCGGCTTCTAGCCCAG

HisThrSerGlyLeuProSerAlaAsnGlyHisAspProGlnAspArgLeuLeuAlaGln

1510 1530 1550

GACTCAGAACACAACCACAGTGACCGGAACTGGCAGCGCAACCGGCCTTGGCCCAAGGAT

AspSerGluHisAsnHisSerAspArgAsnTrpGlnArgAsnArgProTrpProLysAsp

1570 1590 1610

AGCTACTGACAGCCTCCTGCTGCCCTACCCTGGCAGGCACAGGCGCAGCTGGCTGGGGGG

SerTyr

1630 1650 1670

CCCACTCCAGGCAGGGTGGCGTTAGACTGGCATCAGGCTGGCACTAGGCTCAGCCCCCAA

1690 1710 1730

AACCCCCTGCCCAGCCCCAGCTTCAGGGCTGCCTGTGGTCCCAAGGTTCTGGAGAAACA

1750 1770 1790

GGGGACCCCCTCACCTCCTGGGCAGTGACCCCTACTAGGCTCCCATTCCAGGTACTAGCT

1810 1830

GTGTGTTCTGCACCCCTGGCACCGGAATTC

## Patentansprüche

1. cDNA der humanen Beta-Untereinheit spannungsabhängiger Kalziumkanäle der Sequenz 29178-2.

2. Das abgeleitete Protein der in Anspruch 1 genannten cDNA.

3. Die Expression der in Anspruch 1 genannten cDNA in Xenopus Oocyten.

4. Die Expression der in Anspruch 1 genannten cDNA in Insektenzellen.

5. Die Expression der in Anspruch 1 genannten cDNA in Säugerzellen.

6. Die Koexpression der in Anspruch 1 genannten cDNA mit cDNAs der Alpha-1 und Alpha-2-Untereinheit spannungsabhängiger Kalziumkanäle aus verschiedenen Spezies.

7. Die Verwendung der aus Anspruch 6 resultierenden rekombinanten Expressionssysteme als Screeningsystem zur Auffindung und Charakterisierung von Substanzen, welche die Aktivität spannungsabhängiger Kalziumkanäle modulieren.

## Claims

1. cDNA of the human beta subunit of voltage-operated calcium channels with the sequence 29178-2.

2. The protein derived from the cDNA specified in Claim 1.

3. The expression of the cDNA specified in Claim 1 in xenopus oocytes.

4. The expression of the cDNA specified in Claim 1 in insect cells.

5. The expression of the cDNA specified in Claim 1 in mammalian cells.

6. The coexpression of the cDNA specified in Claim 1 with cDNAs of the alpha-1 subunit and alpha-2 subunit of voltage-operated calcium channels from various species.

7. The use of the recombinant expression systems resulting from Claim 6 as screening system for finding and characterizing substances which modulate the activity of voltage-operated calcium channels.

## Revendications

1. ADN complémentaire de la sous-unité bêta humaine de canaux calcium tensio-dépendants de la séquence 29178-2.

2. La protéine dérivée de l'ADN complémentaire mentionné dans la revendication 1.

3. L'expression de l'ADN complémentaire mentionné dans la revendication 1 dans des ovocytes de Xenopus.

4. L'expression de l'ADN complémentaire mentionné dans la revendication 1 dans des cellules d'insectes.

5. L'expression de l'ADN complémentaire mentionné dans la revendication 1 dans des cellules de mammifères.

6. La coexpression de l'ADN complémentaire mentionné dans la revendication 1 avec des ADN complémentaires de la sous-unité alpha-1 et de la sous-unité alpha-2 de canaux calcium tensio-dépendants de différentes espèces.

7. Utilisation des systèmes d'expression recombinants résultant de la revendication 6 comme système de détection pour la mise en évidence et la caractérisation de substances qui modulent l'activité de canaux calcium tensio-dépendants.
